# EUROPEAN PATENT APPLICATION

(11) **EP 4 659 661 A1**
(43) Date of publication of application: **10.12.2025**
(21) Application number: 24186871.0
(22) Date of filing: 05.07.2024
(51) Int. Cl.: A61B 5/0205, A61B 5/024, A61B 5/00

(54) **BABY MONITORING SYSTEM**

(30) Priority: 05.06.2024 WO PCT/CN2024/097592
(71) Applicant: Koninklijke Philips N.V., 5656 AG Eindhoven (NL)
(72) Inventor: LEI, PengFei, Eindhoven (NL); JOOSTEN, Franciscus Ivo, Eindhoven (NL); CLAASSEN, Coen Petrus Martinus, 5656AG Eindhoven (NL); VAN VELDHUIZEN, Gijsbert Hendrik, Eindhoven (NL)
(74) Representative: Philips Intellectual Property & Standards

(57) **Abstract**

A wearable baby monitoring system comprises a baby monitoring unit that comprises a housing with first and casing parts rigidly connected to each other as well as a laminate with at least a first layer bonded to an outer surface of the first casing part and a second layer bonded to an outer surface of the second casing part. In this way, the first and second casing parts are sandwiched within the laminate as well as being rigidly coupled together. This provides two connection systems to increase the security of the housing. An arrangement is for example provided to enable the baby monitoring unit to be worn by a baby.

## Description

### FIELD OF THE INVENTION

The invention relates to baby monitoring systems, in particular with a wearable monitoring unit.

### BACKGROUND OF THE INVENTION

New parents are often faced with a dilemma when putting their infant to bed. On the one hand, they themselves require sufficient rest to recharge, and this means they don't have the time or energy to constantly check on the sleep performance of their infant. On the other hand, they are concerned with how well their infant sleeps, such as the length of the sleep or fears about medical problems such as possible sudden infant death syndrome (SIDS).

The monitoring of sleep and/or vital signs of babies is increasingly popular. Systems for monitoring an infant's heart rate, blood oxygen or breathing rate during sleep have been developed, for example. These systems can be used for non-medical use, but providing consistent, uniform comfortable and dependable monitoring results has proven to be difficult.

There is a need for a consistent, comfortable and dependable monitoring system, that not only can help parents to monitor their baby, such as sleep patterns or other wellness indicators, but also provides them with re-assurance so that they experience peace of mind.

The baby monitoring unit includes hardware sensing components, and to enable consumer use of such components, these are typically enclosed in an enclosure. This enclosure is typically formed by two housing parts that are fixed to each other after the electronic sensing components are placed inside.

For a baby wearable sensor assembly, it is important that the fixation method between the different housing elements cannot come apart. A baby could swallow or choke on a small housing part, or it may come into contact with dangerous electronic components such as a battery.

To lower the risk of accidental disassembly, mitigations need to take place. In particular there is a need for a design of the housing that reduces to a minimum a risk of disassembly of housing parts to reveal the internal components.

### SUMMARY OF THE INVENTION

The invention is defined by the claims.

According to examples in accordance with an aspect of the invention, there is provided a wearable baby monitoring system, comprising:
a baby monitoring unit comprising:
   monitoring hardware;
   a housing enclosing the monitoring hardware, the housing comprising:
      a first casing part; and
      a second casing part, wherein the first casing part and the second casing part are rigidly connected to each other by a first connection;
   a laminate comprising:
      a first layer bonded to an outer surface of the first casing part; and
      a second layer bonded to an outer surface of the second casing part, such that the first and second casing parts are at least partially sandwiched within the laminate to provide a second connection, wherein the first and second layers are bonded together around a perimeter of the housing; and
an arrangement to enable the baby monitoring unit to be worn by a baby.

This system has a monitoring unit which is worn by a baby, by means of an attachment such as a strap arrangement. The monitoring unit houses hardware which is to be kept enclosed. To provide a secure enclosure, two connection methods are used; the first and second casing parts are rigidly connected to each other and also they are sandwiched within a laminate providing an additional, preferably flexible, connection. The laminate partly covers the otherwise exposed external surfaces of the housing. In this way, the risk of accidental disassembly is mitigated by having two means of fixation for the casing parts. The first connection bonds a first rigid part (the first casing part) to a second rigid part (the second casing part) resulting in a very rigid but brittle bond. The second connection bonds the first and second casing parts part to a laminate structure, preferably resulting in a flexible and non-brittle bond.

The first connection may be direct between the first and second casing parts, with no layer of the laminate. This makes it easier to ensure the monitoring unit is waterproof. There may instead be a non-porous layer between them which may also be present within the laminate structure.

The monitoring hardware for example comprises one or both of a PPG sensor and an accelerometer. Thus, the baby monitoring may one or more of monitor heart rate, respiration rate, blood oxygen level, sleep vs. awake, and sleep stages.

The arrangement for example comprises a web with an opening through which the housing projects, wherein the laminate is releasably attached to the web. Thus, the arrangement is in the form of a strap for fitting to the baby, and it holds the monitoring unit against the baby, by holding down the monitoring unit around the housing, i.e., by having the web press down on the laminate which extends around the housing.

The arrangement for example comprises a foot strap.

The first casing part for example comprises a top part and the second casing part comprises a base part, wherein the base part is for mounting against the skin.

A side wall for example extends around the top part, and the first layer is bonded to the side wall. Thus, the laminate connects to the bottom part (e.g. to the underside of the bottom part) and around the side wall of the top part. The laminate could cover the top part, or it may leave the top surface of the top part exposed, projecting through an opening in the laminate.

The base part for example has a bottom wall, and the second layer is bonded to the bottom wall. The second layer for example has an interrogation window which is used by the hardware to perform a monitoring function, such as an optical monitoring function, e.g. optical PPG.

The first and second casing parts are for example bonded together. This is one option for the rigid connection. The first and second casing parts may additionally comprise a mechanical interlocking. They may for example be a snap fit together, with additional bonding. Alternatively (or additionally), the rigid connection may use screws or other fastenings.

The first and second layers comprise fabric layers.

The laminate may further comprise a third layer bonded to the second layer, such that the second and third layers are bonded to the second casing part. This third layer for example enables a more even skin contacting surface to be achieved.

The laminate may further comprise a fourth layer between the first and second casing parts. This fourth layer is for example a non-porous layer such as a thin plastic film which is glued between them. Suich a plastic film is for example used as a base on which layers of the laminate are glued outside the regions where the first and second casing parts are directly connected.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a wearable baby monitoring system;
Fig. 2 shows the monitoring unit in side view.
Fig. 3 shows one example of the different parts of the monitoring unit in exploded view.
Fig. 4 shows another example of the different parts of the monitoring unit 10 in exploded view; and
Fig. 5 shows a simplified cross section of the monitoring unit corresponding to the example of Fig. 2.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a wearable baby monitoring system that comprises a baby monitoring unit that comprises a housing with first and casing parts rigidly connected to each other as well as a laminate with at least a first layer bonded to an outer surface of the first casing part and a second layer bonded to an outer surface of the second casing part. In this way, the first and second casing parts are sandwiched within the laminate as well as being rigidly coupled together. This provides two connection systems to increase the security of the housing. An arrangement is for example provided to enable the baby monitoring unit to be worn by a baby.

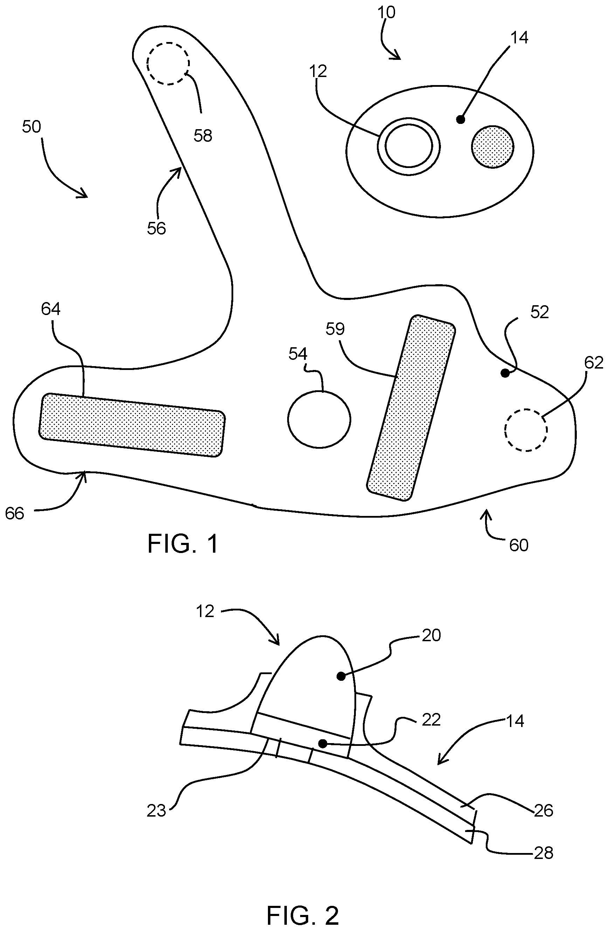

Fig. 1 shows a wearable baby monitoring system, comprising a baby monitoring unit 10 and an arrangement 50 to enable the baby monitoring unit 10 to be worn by a baby.

The arrangement 50 comprises a strap system, and in the example shown it is for wrapping around the foot of a baby. The arrangement 50 comprises a web 52 with an opening 54. The web has a first strap 56 with a Velcro (TM) patch 58 (on the opposite side to that shown). The first strap wraps around the ankle and then connects with Velcro patch 59 (which will be at the top of the foot). A second strap 60 has another Velcro patch 62 (on the opposite side to that shown). The second strap 60 wraps under the foot and then connects with Velcro patch 64 on strap 66.

Of course, other releasable fastenings may be used. Thus, the arrangement has straps to attach to a foot by wrapping around the foot and ankle. The opening 54 is positioned on top of the foot, for example to be positioned over a vein.

The monitoring unit comprises a housing 12 for holding monitoring hardware. The housing 12 stands upwardly from a laminate 14, preferably a flexible laminate.. The monitoring unit is positioned under the arrangement 50 against the baby's skin, and the housing 12 projects through the opening 54. Thus, the arrangement 50 holds the monitoring unit 10 against the skin by pressing down on the laminate 14. The monitoring unit also has a Velcro patch 16 to enable attachment to the underside of the arrangement 50.

In another example, the arrangement for against the baby's skin may be an integral part of the monitoring unit.

Fig. 2 shows the monitoring unit in side view.

The housing 12 comprises a first casing part 20 and a second casing part 22. The first casing part comprises a top part and the second casing part comprises a base part, and the base part is for mounting against the skin. In other designs, the casing may be split in a plane which is not parallel to the base.

When connected, they define a chamber for monitoring hardware. The monitoring hardware for example comprises one or both of a PPG sensor and an accelerometer. Thus, the baby monitoring may one or more of monitor heart rate, respiration rate, blood oxygen, sleep vs. awake, and sleep stages. Any desired monitoring sensors may be housed in the housing.

The first casing part and the second casing part are rigidly connected to each other by a first connection.

The flexible laminate 14 comprises a first layer 26 bonded to an outer surface of the first casing part 20. A second layer 28 of the flexible laminate is bonded to an outer surface of the second casing part 22, such that the first and second casing parts are at least partially sandwiched within the flexible laminate to provide a second, flexible, connection. The second layer 28 is bonded to a bottom wall 23 of the second casing part 22.

The first and second layers 26, 28 are bonded together around a perimeter of the housing. They comprise fabric layers to provide flexibility and comfort.

Two connections are thus used to provide a secure enclosure. Firstly, the first and second casing parts 20, 22 are rigidly connected to each other. This may be achieved by gluing, by a mechanical coupling (such as a snap fit), by fastenings such as screws, or by a combination of these. Secondly, the casing parts are sandwiched within a laminate 14 providing an additional flexible connection. In this way, the risk of accidental disassembly is mitigated by having two means of fixation for the casing parts.

The first connection may be direct between the first and second casing parts 20, 22, with no layer of the flexible laminate (as is shown in Fig. 2). This makes it easier to ensure the monitoring unit is waterproof, because the flexible layers of the laminate are typically porous. However, there may be a non-porous layer bonded between the casing parts 20, 22, for example to assist in providing a secure bond.

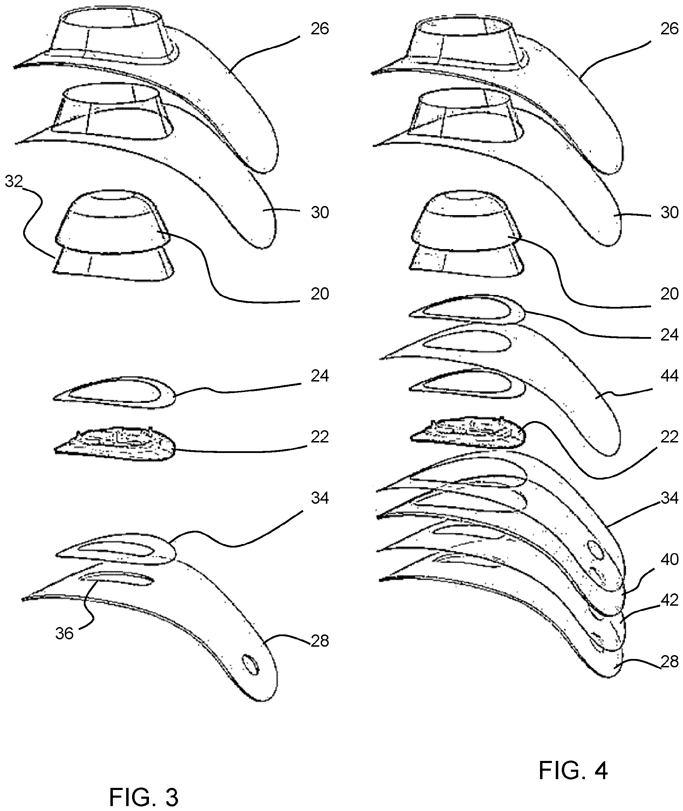

Fig. 3 shows one example of the different parts of the monitoring unit 10 in exploded view.

The first casing part 20 and the second casing part 22 are rigidly connected to each other by the first connection which is shown as a glue layer 24.

The first layer 26 of the flexible laminate is bonded to an outer surface of the first casing part 20 by glue layer 30. The first layer 26 could over the first casing part 20, but in the example shown, the first casing part 20 projects through the laminate (as is also shown in Fig. 2). The first layer 26 is bonded by the glue layer 30 around an upstanding side wall 32 of the first casing part.

The second layer 28 is bonded by the glue layer 34 to a bottom wall of the second casing part 22. The second layer 28 for example has an interrogation window 36 which is used by the hardware to perform a monitoring function, such as an optical monitoring function.

The first casing part 20 has a mushroom head shape so that it is pushed through openings in the first layer and glue layer, and this simplifies the assembly. The first layer is glued to the side wall 32 around the stem of the mushroom head shape.

Fig. 4 shows another example of the different parts of the monitoring unit 10 in exploded view.

The same parts as in Fig. 3 are given the same reference numbers.

A third layer 40 is added, bonded to the second layer 28, such that the second and third layers 28, 40 are bonded to the second casing part by glue layers 34, 42 (an additional glue layer 42 is provided compared to Fig. 3). This third layer 40 for example enables a more even skin contacting surface to be achieved. It comprises another fabric layer, so that the laminate has a top fabric layer 26, a middle fabric layer 40 and a bottom fabric layer 28.

A fourth layer 44 is added between the first and second casing parts 20, 22. This fourth layer is for example a non-porous layer such as a thin plastic film which is glued between the casing parts by glue layers 24, 46 (an additional glue layer 46 is provided compared to Fig. 3). Such a plastic film is for example used as a base on which layers of the laminate are glued outside the regions where the first and second casing parts are directly connected.

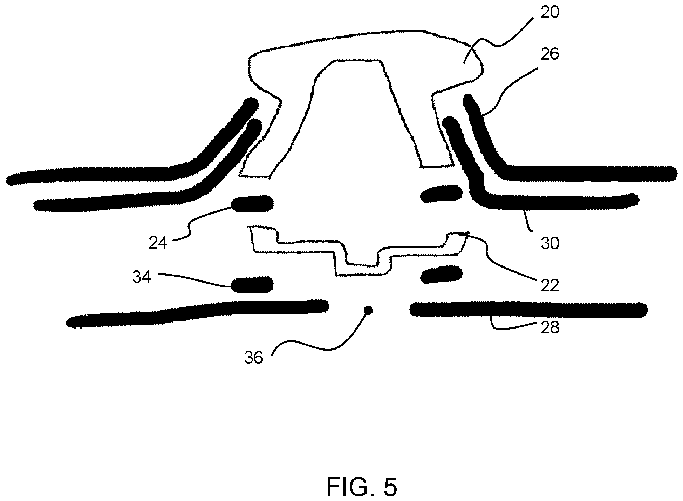

Fig. 5 shows a simplified cross section of the monitoring unit corresponding to the example of Fig. 2.

The layers between the casing parts have openings so that they do not intrude into the housing cavity. The laminate layers beneath the second casing part also have openings to function as the interrogation window.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A wearable baby monitoring system, comprising:
a baby monitoring unit (10) comprising:
monitoring hardware;
a housing (12) enclosing the monitoring hardware, the housing comprising:
a first casing part (20); and
a second casing part (22), wherein the first casing part and the second casing part are rigidly connected to each other by a first connection (24);
a laminate (14) comprising:
a first layer (26) bonded to an outer surface of the first casing part (20); and
a second layer (28) bonded to an outer surface of the second casing part (22), such that the first and second casing parts are at least partially sandwiched within the laminate to provide a second connection, wherein the first and second layers (26, 28) are bonded together around a perimeter of the housing.

2. The system of claim 1, wherein the laminate is flexible such that the second connection is flexible.

3. The system of claim 1 or 2, wherein the monitoring hardware comprises one or both of a PPG sensor and an accelerometer.

4. The system of any one of claims 1 to 3, further comprising an arrangement (50) to enable the baby monitoring unit to be worn by a baby.

5. The system of claim 4, wherein the arrangement comprises a web (52) with an opening (54) through which the housing (12) projects, wherein the laminate is releasably attachable to the web.

6. The system of claim 4 or 5, wherein the arrangement (50) comprises a foot strap.

7. The system of any one of claims 1 to 6, wherein the first casing part (20) comprises a top part and the second casing part (22) comprises a base part, wherein the base part is for mounting against the skin.

8. The system of claim 7, wherein a side wall (32) extends around the top part, and the first layer (26) is bonded to the side wall.

9. The system of claim 7 or 8, wherein the base part has a bottom wall (23), and the second layer (28) is bonded to the bottom wall (23).

10. The system of claim 9, wherein the second layer has an interrogation window (36).

11. The system of any one of claims 1 to 10, wherein the first and second casing parts (20, 22) are bonded together.

12. The system of claim 11, wherein the first and second casing parts (20, 22) additionally comprise a mechanical interlocking.

13. The system of any one of claims 1 to 11, wherein the first and/or second layers (26, 28) comprise fabric layers.

14. The system of any one of claims 1 to 13, wherein the laminate further comprises a third layer (40) bonded to the second layer (28), such that the second and third layers are bonded to the second casing part (22).

15. The system of claim 14, wherein the laminate further comprises a fourth layer (44) between the first and second casing parts (20,22).
